# EUROPEAN PATENT SPECIFICATION

(11) **EP 4 026 495 B1**
(45) Date of publication and mention of the grant of the patent: **04.11.2026**
(21) Application number: 20859762.5
(22) Date of filing: 12.06.2020
(51) Int. Cl.: A61B 5/145, A61B 5/00, A61B 5/1455, A61B 10/00, H04M 1/21, H04M 1/725, G02B 3/00, G02B 5/08, A61B 5/15

(54) **BODY FLUID TESTER**
KÖRPERFLUIDTESTER
TESTEUR DE LIQUIDE CORPOREL

(30) Priority: 02.09.2019 KR 20190108058
(43) Date of publication of application: 13.07.2022
(73) Proprietor: INTIN INC., Daegu 41069 (KR)
(72) Inventor: KIM, Ji Hoon, Seoul 18449 (KR)
(74) Representative: Sander, Rolf
(86) International application number: PCT/KR2020/007652
(87) International publication number: WO 2021/045359

(56) References cited:
- WO-A1-2018/079915
- WO-A1-2018/097565
- WO-A1-2018/166533
- JP-A- 2016 163 722
- KR-A- 20170 071 277
- KR-A- 20180 057 306
- KR-A- 20180 057 308
- KR-U- 20000 015 504
- US-A1- 2016 080 548
- US-A1- 2017 119 357
- US-A1- 2018 141 041
- US-B2- 9 787 815

## Description

### BACKGROUND OF THE DISCLOSURE

### Field of the disclosure

The present disclosure relates to a body fluid tester, and more particularly, to a body fluid tester which allows a carrier, on which a test sheet with body fluid received therein is mounted, to be easily separated from a main body of the body fluid tester.

### Related Art

Generally, people should visit a specialized medical institution to check there health state and body state. However, in order to receive diagnosis, people need to set aside time for visiting a specialized medical institution during busy work time, and it takes a complicated procedure.

In particular, this procedure requires a long wait to receive an examination, but the actual examination or inspection is conducted in a very short time. That is, it takes a lot of time and money to receive the examination or inspection, which involves a lot of inconvenience.

Thus, most people ignore endurable discomfort, and visit specialized medical institutions only when having unendurable pain or discomfort.

In order to solve the inconvenience, techniques have been developed wherein various types of advanced equipment are used to frequently check one's body state and the result is transmitted to a specialized medical institution via a network. However, techniques that are commercialized and are actually used in practice are extremely rare.

Preferably, the use of a simple tester, which has been used for a long time, continues and is increasingly used by the younger generation due to ease of use, low cost, and convenience.

Such a tester detects biochemical substances such as hormones discharged under particular circumstances by using secretions from people's bodies, e.g., saliva, urine, sweat, etc. The tester may be used to detect hormones included in the biochemical substances or to check a change in state of the biochemical substances.

The test device uses a change in the composition of saliva or a change in state when the body is subjected to a specific situation, for example, a specific disease, infection, or abnormality or peculiarity of the body such as ovulation.

As a commonly used testing means, testers for pregnancy testing, diabetes diagnosis, and blood sugar checking are used. In particular, the tester is relatively highly accurate, easy to use, and inexpensive, and thus, many people widely use the tester.

Recently, due to increase in the age at marriage, stress, lack of exercise, etc., the number of couples who are infertile and have difficulty in becoming pregnant has increased, and thus the use of pregnancy tester has greatly increased. However, the existing pregnancy tester may determine only pregnancy or not and is not helpful for the user who is infertile and has difficulty in becoming pregnant.

Furthermore, only the woman's pregnancy can be identified by the exiting pregnancy tester, and the woman's pregnancy probability, ovulation, the man's fertilization ability, etc. can be identified only by a medical institution's consultation.

US 2016/080548 A1 discloses a method for obtaining a point-of-collection, selected quantitative indicia of an analyte on a test strip using a smartphone involves imaging a test strip on which a colorimetric reaction of a target sample has occurred due to test strip illumination by the smartphone. The smartphone includes a smartphone app and a smartphone accessory that provides an external environment-independent/internal light-free, imaging environment independent of the smartphone platform being used.

WO 2018/097565 A1 discloses a testing device having improved illumination, which provides illumination so as for a test to be conducted at any time regardless of the environment, obtains a clear image of a body fluid, and allows the test to be conducted accurately.

### SUMMARY

The present disclosure provides a body fluid tester. More particularly, the present disclosure provides a body fluid tester which allows a carrier, on which a test sheet with body fluid received therein is mounted, to be separated from a main body of the body fluid tester.

In an aspect, a body fluid tester is provided, according to the invention as defined by claim 1. Further advantageous embodiments of the present invention are defined by the dependent claims. The body fluid tester includes: a carrier having a sheet insertion hole, the sheet insertion hole being configured to receive a test sheet by insertion in a first horizontal direction; an outer case having a carrier hole formed in a front side thereof, the carrier hole which has its length in a second horizontal direction perpendicular to the first horizontal direction and intersecting with the first horizontal direction and into which the carrier is configured to be inserted, the outer case having an opened lower side; an inner case configured to be inserted upwards through a lower side of the outer case following a vertical direction perpendicular to the horizontal directions to be coupled thereto positioned at an inner surface of the outer case, and having a case observation hole at a bottom side thereof; and a lens unit positioned on a side of an inner front end of the inner case and positioned between the carrier and the case observation hole. The carrier is configured to be inserted into or separated from the carrier hole of the outer case.

Here, the carrier includes: a head part having a first thickness and exposed to a front of the outer case; and a body part having a second thickness smaller than the first thickness, extending from the head part in the first horizontal direction, and having a through hole opened in the vertical direction intersecting with the first and second horizontal directions. The through hole of the carrier, the lens unit, and the case observation hole of the inner case are configured to be aligned along a vertical optical path when the carrier is fully inserted into the outer case.

A maximum width of the head part in the second horizontal direction may be greater than a minimum width of an opening hole of the outer case in the second horizontal direction.

The body part may include: a pair of upper protrusions provided at an upper side of the body part, protruding from the head of the carrier in the first horizontal direction, spaced apart from each other at a first interval, and having a first width; and a pair of lower protrusions provided at a lower side of the body part, protruding from the head part of the carrier in the first horizontal direction, spaced apart from each other at a second interval, and having a second width.

A coupling protrusion may be provided in an inner surface of the outer case, the coupling protrusion protruding downward to be inserted between the pair of the upper protrusions, and a pair of inner coupling holes may be provided at a front upper end of the inner case, the inner coupling holes into which the pair of lower protrusions are to be inserted.

A first height difference between the head part and the body part at an upper side of the carrier may be different from a second height difference between the head part and the body part at a lower side of the carrier.

The first interval between the pair of upper protrusions may be different from the second interval between the pair of lower protrusions, or a first width between the pair of upper protrusions may be different from a second width between the pair of lower protrusions.

The lens unit may include: an outer housing having a length in a vertical direction; and an inner housing positioned within the outer housing, having a length in the vertical direction, and having a plurality of lenses spaced apart from each other in the vertical direction, and wherein the outer housing has a screw groove provided in an inner surface thereof. The inner housing may have a screw groove provided in an outer surface thereof to be engaged with the screw groove of the outer housing.

In addition, the body fluid tester may further include: a light source provided above the carrier within the outer case; and a diffusing lens provided above the light source to diffuse light emitted from the light source.

The body fluid tester may further include an upper light hole provided at an upper side of the outer case, the upper light hole through which the light emitted from the light source passes.

The test sheet may include a body fluid receiver, and the body fluid receiver may be provided in a shape of a groove recessed from an upper side of the test sheet, or the body fluid receiver may be provided by forming a partition wall at the upper surface of the test sheet.

In addition, the body fluid tester may further include a main body fixing part separable from a bottom of the inner case and having a recessed groove, and a fixing part observation hole may be provided in a surface in contact with a case observation hole of the inner case in a bottom of the recessed groove and a magnetic body is provided in at least a portion of the remaining part of the bottom of the recessed groove.

In the body fluid tester according to an exemplary embodiment of the present disclosure, the carrier is allowed to be inserted into or separated from the carrier hole of the outer case, so that a user can easily wash the carrier.

In the body fluid tester according to an example of the present disclosure, the upper side and the lower side of the carrier may be easily distinguished, so that the user can use a body fluid examination device more conveniently.

### BRIEF DESCRIPTION OF THE DRAWINGS

FIG. 1 is a view for explaining an example of using a body fluid tester according to an example of the present disclosure.
FIG. 2 is a view showing an external appearance of a body fluid tester according to an example of the present disclosure.
FIGS. 3 and 4 are views for explaining a structure of the body fluid tester shown in FIGS. 1 and 2 according to an example of the present disclosure.
FIG. 5 is a view for explaining a test sheet inserted into a body fluid tester of the present disclosure.
FIG. 6 is a view for explaining a configuration that operates when the test sheet of FIG. 5 is inserted into a main body of a body fluid tester.
FIGS. 7A and 7B are views for explaining in more detail a carrier in the present disclosure.
FIG. 8 is a view for explaining a coupling protrusion of an outer case coupled to the carrier and an inner coupling hole of an inner case.
FIG. 9 is a view for explaining a comparison between a width of the carrier in a second horizontal direction and a width of a carrier hole of the outer case in the second horizontal direction.
FIG. 10 illustrates a lens unit of a body fluid tester according to an example of the present disclosure in more detail.
FIG. 11 is a view for explaining a modified example in which a part of a body fluid tester according to the present disclosure shown in FIGS. 3 and 4 is changed.
FIG. 12 is a view for explaining a main body fixing part in a body fluid tester according to an example of the present disclosure.

### DESCRIPTION OF EXEMPLARY EMBODIMENTS

Hereinafter, embodiments of the present disclosure will be described in detail with reference to the accompanying drawings. In describing the present disclosure, if it is determined that a detailed description of known functions and components associated with the present disclosure. The terms used henceforth are used to appropriately express the embodiments of the present disclosure and may be altered according to a person of a related field or conventional practice. Therefore, the terms should be defined on the basis of the entire content of this specification.

Hereinafter, a body fluid tester according to the present disclosure will be described with reference to the accompanying drawings.

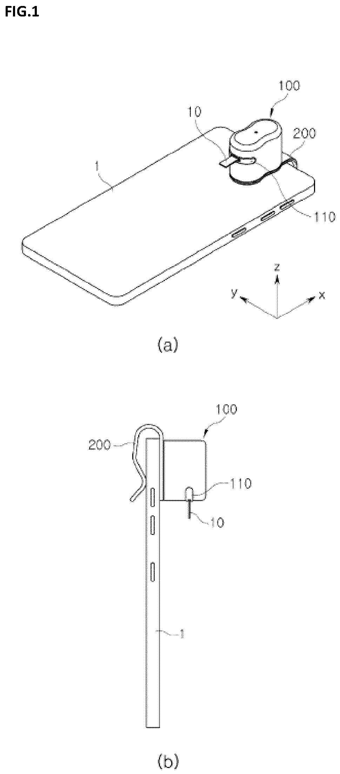

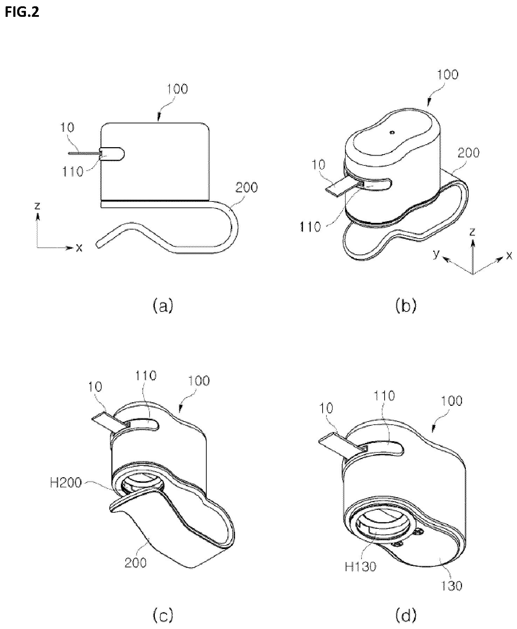

FIG. 1 is a view for explaining an example of using a body fluid tester according to an example of the present disclosure, and FIG. 2 shows an external appearance of a body fluid tester according to an example of the present disclosure.

In FIG. 1, (a) is a perspective view of an example of using a body fluid tester, and (b) shows a side view of an example of using a body fluid tester.

A body fluid tester according to an example of the present disclosure greatly enlarges body fluid such as saliva (or spit), vaginal discharge, semen, saliva, urine, and sweat, so that a user can easily and conveniently analyze the body fluid.

Such a body fluid tester of the present disclosure may be used in combination with a user terminal 1 as shown in (a) and (b) of FIG. 1, but this is merely an example, and a user is also able to observe body fluid with bare eyes. Hereinafter, for convenience of use and analysis, a case in which a body fluid tester is used in combination with the user terminal 1 will be described as an example.

In the user terminal 1, a body fluid analysis program or application for analyzing video image information acquired through a body fluid tester may be installed.

For example, if the user terminal is a device capable of performing communication, the user terminal 1 may include both wired and wireless communication devices and may include a mobile phone, a smart phone, a tablet computer, a laptop computer, a portable media player, and a Personal Digital Assistant (PDA), a wearable device such as a smart watch or smart glasses capable of wireless communication, and a navigation device.

However, even if the body fluid analysis program or application is not installed in the user terminal 1, image information acquired by a camera of the user terminal 1 may be transmitted to another electronic communication device in which the body fluid analysis program or application is installed.

As shown in FIG. 2, a body fluid tester according to an exemplary embodiment of the present disclosure includes a carrier 110 into which a test sheet 10 with body fluid received therein is inserted, a main body 100 of the body fluid tester in which the carrier 110 is mounted, and a main body fixing part 200 for coupling the main body 100 of the body fluid tester to the user terminal 1.

The body fluid tester may be attached to the user terminal 1 and provide an enlarged image of body fluid so that the user terminal 1 may capture and analyze an image of the body fluid by using the body fluid as a sample.

More specifically, in the body fluid tester according to an example of the present disclosure, as the test sheet 10 with body fluid received therein is be inserted into the carrier 110 provided in the main body 100 of the body fluid tester, a light source 170 in the main body 100 of the body fluid tester may emit to radiate light onto the test sheet 10 and the body fluid in the test sheet 10 onto which the light is radiated may be captured by a camera to acquire an image of a body fluid sample.

As such, in order for the camera of the terminal 1 to capture an image of the body fluid, a fixing part observation hole H200 and a case observation hole H130 may be provided in the body fluid tester and the main body fixing part 200, respectively, as shown in FIG. 2.

Hereinafter, an internal structure of the body fluid tester will be described in more detail.

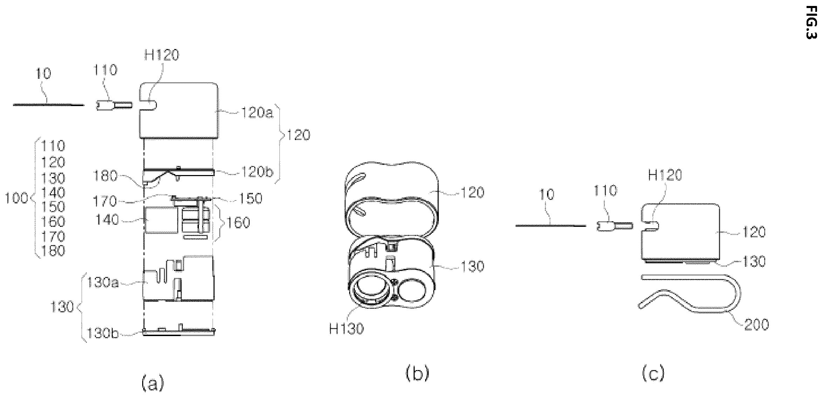

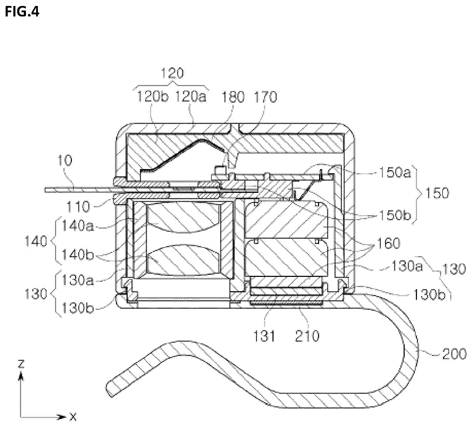

FIGS. 3 and 4 are views for explaining a structure of the body fluid tester shown in FIGS. 1 and 2 according to an example of the present disclosure.

(a) of FIG. 3 shows a state in which the test sheet 10, the carrier 110, each element of the main body 100 of the body fluid tester, and the main body fixing part 200 are separated; (b) of FIG. 3 shows a state in which an outer case 120 is separated from the body fluid tester; (c) of FIG. 3 shows a state in which the test sheet 10, the carrier 110, the main body 100 of the body fluid tester, and the main body fixing part 200 are separated; and FIG. 4 shows a cross-section in which the test sheet 10, the carrier 110, each element of the main body 100 of the body fluid tester, and the main body fixing part 200 are coupled.

As shown in FIGS. 3 and 4, the body fluid tester according to an example of the present disclosure may include a main body 100 of the body fluid tester and a main body fixing part 200. The body 100 of the body fluid tester may include the carrier 110, the outer case 120, an inner case 130, a lens unit 140, a driving board of a driver 150, a power supply 160, the light source 170, and a reflection unit 180.

Here, the carrier 110 and the main body fixing part 200 may be detachably attached to the main body 100 of the body fluid tester.

In the body fluid tester of the present disclosure, the test sheet 10, the carrier 110, and the main body fixing part 200 may be separated, as shown in (c) of FIG. 3.

More specifically, when the body fluid tester is in use, the test sheet 10 and the carrier 110 may be used in combination with the main body 100 of the body fluid tester, or the test sheet 10, the carrier 110, and the main body fixing part 200 may be used in combination with the main body 100 of the body fluid tester.

When the body fluid tester is not in use, the test sheet 10, the carrier 110, and the main body fixing part 200 may be separated from the main body 100 of the body fluid tester. In particular, the carrier 110 into which the test sheet 10 is inserted is detachable from the main body 100 of the body fluid tester, thereby providing convenience for a user to easily wash the carrier 110.

As such, in the body fluid tester according to an embodiment of the present disclosure, the carrier 110 may be inserted into or detached from the carrier hole H120 of the outer case 120 and thereby a user is able to separate and detach the carrier 110 from the main body 100 of the body fluid tester to wash. Accordingly, user convenience may be further improved to use the body fluid tester more cleanly.

The main body 100 of the body fluid tester may serve as an objective part for allowing the user's body fluid to be used as a sample to be analyzed by the user terminal 1.

That is, the main body 100 of the body fluid tester may be coupled to the user terminal 1 by the main body fixing part 200, and may be coupled in a manner in which the body fluid used as a sample is applied on the test sheet 10.

Accordingly, the test sheet 10, the lens unit 140 provided in the main body 100 of the body fluid tester, and the camera of the user terminal 1 may be aligned in a vertical direction z, and a state of the body fluid magnified approximately 300 times to 1000 times by the lens unit 140 provided in the main body 100 of the body fluid tester may be captured by the user terminal 1.

In addition, the user terminal 1 may analyze an image of the body fluid during a processing process of a pre-installed program to determine the user's physical condition.

The body 100 of the body fluid tester may be detachably attached to the user terminal 1 by the main body fixing part 200 as shown in FIG. 3.

Specifically, the main body fixing part 200 may be formed in a clip shape. The body fixing part 200 may be coupled to the main body 100 of the body fluid tester, but may be easily removed therefrom, as shown in (a) of FIG. 3.

Specifically, the main body 100 of the body fluid tester and the main body fixing part 200 may be detachably attached using a means that can be repeatedly attached and detached, such as a magnet.

The main body fixing part 200 may couple the main body 100 of the body fluid tester to the user terminal 1 and may allow the case observation hole H130 of the main body 100 of the body fluid tester to match with the camera of the user terminal 1. To this end, the main body fixing part 200 may be provided with the fixing part observation hole H200, and may be formed of a shape and a material having elasticity.

The main body 100 of the body fluid tester may be coupled by the user terminal 1 and the main body fixing part 200 to provide lighting and a magnification means for body fluid applied to the test sheet 10, so that the user terminal 1 can acquire an image of the body fluid using the lighting and the magnification means.

When the test sheet 10 coated with the body fluid as described above is inserted, the main body 100 of the body fluid tester operates to provide light, so that a clear image of the body fluid can be captured through the camera of the user terminal 1.

To this end, as the test sheet 10 is inserted into and coupled to the main body 100 of the body fluid tester 100, the main body 100 of the body fluid tester may allow light to be radiated onto a body fluid sample seated on the test sheet 10 so that the body fluid sample seated on the test sheet 10 can be magnified and observed through a plurality of lenses,

To this end, the main body 100 of the body fluid tester (hereinafter, referred to as the main body 100) according to an example of the present disclosure includes a carrier 110, an outer case 120, an inner case 130, a lens unit 140, a driver 150, a power supply 160, a light source 170, and a reflection unit 180.

As shown in FIG. 4, a test sheet 10 with the body fluid received therein is inserted, according to the invention, into the carrier 110, and the test sheet 10 may be inserted into a carrier hole H120 of the main body 100 and thereby coupled to the main body 100, and accordingly, the carrier 110 can help the test sheet 10 to be easily guided into the main body 100.

The carrier 110 is inserted into the carrier hole H120 provided in the main body 100 after the test sheet 10 is inserted into the carrier 110, or the test sheet 10 may be inserted into the carrier 110 in a state in which the carrier 110 is inserted into the carrier hole H120 of the main body 100.

As such, when the test sheet 10 is inserted into the main body 100, an end of the test sheet 10 may be inserted into the driver 150, thereby operating the driver 150, so that the light source 170 can emit light.

Accordingly, in order for the test sheet 10 with the body fluid received therein to be inserted into the carrier 110 and for the camera of the user terminal 1 to capture the body fluid from the rear side of the carrier 110, a through hole is provided in the carrier 110 so that the body fluid on the test sheet 10 can be exposed in a vertical direction z. The specific structure of the carrier 110 will be described later.

The carrier 110 may be formed of a transparent or translucent synthetic resin material or a plastic material. As such, since the carrier 110 is formed of a transparent or translucent material, when the test sheet 10 is inserted and the light source 170 provided in the main body 100 emits light, a user may be able to easily recognize the light from the light source 170, which is wave-guided along the carrier 110, from the outside of the main body 100.

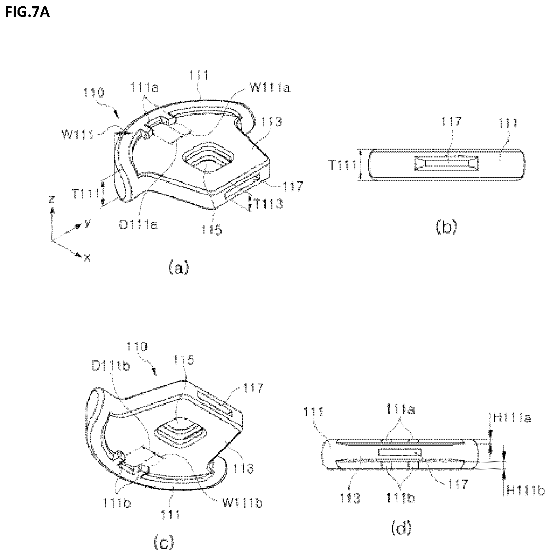

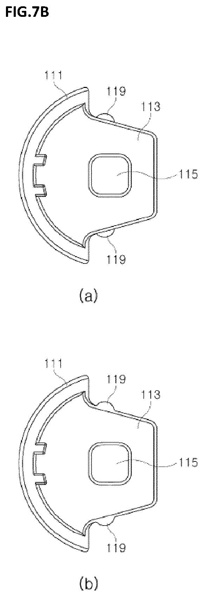

According to the invention, the carrier 110 has a structure in which the test sheet 10 with the body fluid received therein is easily inserted into or separated from a sheet insertion hole and the carrier hole H120 of the main body 100 in a first horizontal direction x. The specific structure of the carrier 110 will be described in detail below with reference to FIG. 7.

The outer case 120 may form an outer appearance of the main body 100 and protect the main body 100 from an external impact or contamination. According to the invention, the outer case 120 has the carrier hole H120 formed in a front side thereof, the carrier hole H120 which has a length in a second horizontal direction y intersecting with the first horizontal direction x and into which the carrier 110 is inserted, and the outer case 120 has an opened lower side.

The outer case 120 may be formed of a mechanically strong material such as metal or synthetic resin, and may be coupled to the inner case 130 to accommodate various components provided therein.

To this end, the outer case 120 may include a first outer member 120a forming an outer appearance of the main body 100, and a second outer member 120b provided inside an upper part of the first outer member 120a and having the reflection unit 180 mounted therein. The first and second external members of the outer case 120 may be formed as separate components or may be formed integrally.

According to the invention, the inner case 130 is inserted upwards through a lower side of the outer case 120 to be coupled thereto so as to be positioned at an inner surface of the outer case 120, and the inner case 130 has a case observation hole H130 at a bottom.

The inner case 130 may be coupled to the inside of the outer case 120 to provide a space in which the lens unit 140, the power supply 160, and the driver 150 are fixed and accommodated.

To this end, the inner case 130 may include a first inner member 130a providing a space in which the lens unit 140, the power supply 160, and the driver 150 are fixed and accommodated, and a second inner member 130b positioned at a bottom of the first inner member 130a, fixed to the outer case 120, and provided with the case observation hole H130. The first and second inner members of the inner case 130 may be formed as separate components or may be formed integrally.

Here, a height of a portion in which the lens unit 140 is accommodated in the first inner member 130a may be different from a height of a portion in which the power supply 160 and the driver 150 are accommodated.

For example, the height of the portion in which the lens unit 140 is accommodated in the first inner member 130a may be relatively lower than the height of the portion in which the power supply 160 and the driver 150 are accommodated, and an end of the portion in which the lens unit 140 is accommodated in the first inner member 130a may overlap with a position of the carrier hole H120 provided in the outer case 120 in the first horizontal direction x.

Accordingly, the driver 150 into which the test sheet 10 is inserted is positioned above the lens unit 140, so that an end of the test sheet 10 inserted into the driver 150 can be naturally positioned above the lens unit 140.

Although an example in which the inner case 130 is provided separately from the outer case 120 for convenience of manufacturing has been described above, the present disclosure is not necessarily limited thereto and the outer case 120 and the inner case 130 may be formed integrally.

In addition, a metal member 131 including a metal material for attachment and detachment with the main body fixing part 200 may be further provided between an upper side of the second inner member 130b and the power supply 160 in the inner case 130.

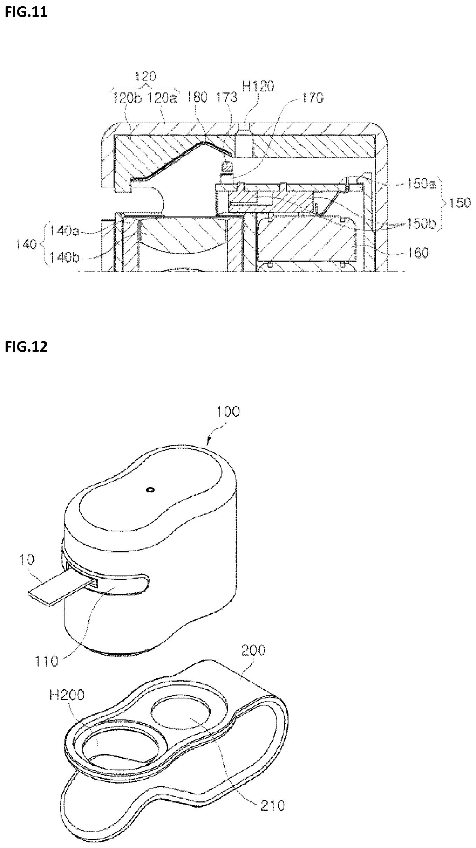

In addition, the main body fixing part 200 may be provided with a magnetic body 210 for attachment and detachment with the main body 100. A more specific structure of the main body fixing part 200 will be described in more detail with reference to FIG. 12.

The lens unit 140 may be positioned on the side of an inner front end of the inner case 130 while positioned between the carrier 110 in which the test sheet 10 is inserted and the case observation hole H130, thereby enabled to magnify body fluid seated on the test sheet 10.

The lens unit 140 may include a plurality of lenses, the carrier 110 may be positioned above the lens unit 140, and the case observation hole H130 may be positioned below the lens unit 140. Accordingly, the test sheet 10 may be positioned at a predetermined focal length from the lens unit 140.

Accordingly, the user terminal 1 positioned below the lens unit 140 may magnify and capture a body fluid sample seated on the test sheet 10 of the carrier 110 through the lens unit 140.

The driver 150 may include a circuit pattern for emitting light from the light source 170, and may cause the light source 170 to receive power from the power supply 160 to operate.

Thus, according to the present disclosure, when a user observes the body fluid sample through the observation hole of the main body 100 with naked eyes or the user terminal 1, it is possible to acquire a clearer image.

As shown in FIG. 4, the driver 150 may allow the light source 170 to naturally emit light in response to insertion of the end of the test sheet 10, thereby further improving user convenience.

The driver 150 may be coupled to the inner case 130 to be positioned above an inner rear end of the first inner member 130a of the inner case 130 and positioned at an upper end of the power supply 160, and may be electrically connected through a lead wire of the power supply 160 to receive power.

Although the light source 170 is positioned above the driver 150 and the power supply 160 is positioned below the driver 150, the present disclosure is not necessarily limited thereto.

The power supply 160 may be positioned below the driver 150 at an inner rear end of the inner case 130 to supply power to the driver 150. To this end, a plurality of batteries may be accommodated in the power supply 160.

The light source 170 may be positioned on the driver 150, and upon operation of the driver 150, the light source 170 may receive power from the power supply 160 to emit light. To this end, the light source 170 may include, for example, a light emitting device such as a light emitting diode, and may emit monochromatic light or white light.

The reflection unit 180 may be positioned on the light source 170 while coupled to the upper inner side of the outer case 120, and may receive light emitted from the light source 170 and reflect the received light in a direction toward the test sheet 10.

In doing so, the reflection unit 180 may reflect and scatter the light emitted from the light source 170, so that the light can be radiated to the test sheet 10. In doing so, light is provided evenly to the test sheet 10 so that the camera of the user terminal 1 can acquire a clear image of the body fluid.

The reflection unit 180 may have at least one curved surface depending on an installation position thereof and a positional relationship with the light source 170. The reflection unit 180 may be formed of metal or glass, but the material of the reflection unit 180 is not limited thereto. In addition, a surface of the reflection unit 180 may be processed to have concave-convex portions for light scattering.

In the body fluid tester according to an example of the present disclosure, when the test sheet 10 is inserted into the main body 100, light may be emitted from the inside of the main body 100 and radiated onto the test sheet 10, so that a user can more easily to observe and analyze the body fluid.

Hereinafter, the test sheet 10 inserted into the body fluid tester according to the present disclosure will be described in more detail.

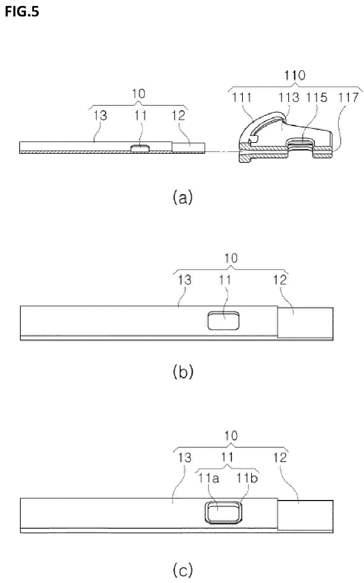

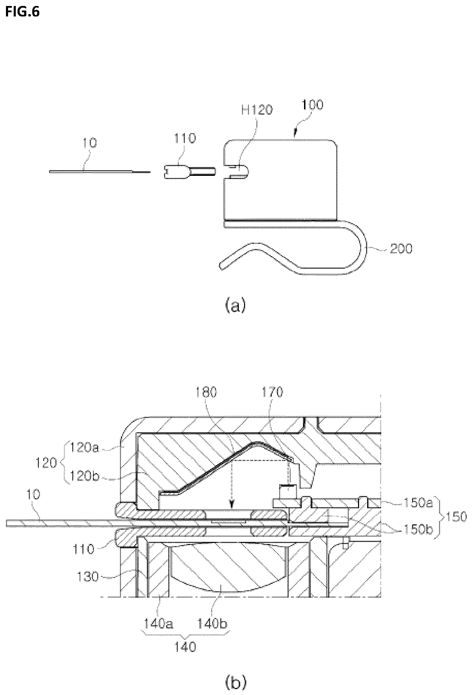

FIG. 5 is a diagram for explaining a test sheet 10 inserted into a body fluid tester of the present disclosure, and FIG. 6 is a diagram for explaining a operation when the test sheet 10 of FIG. 5 is inserted into a main body 100 of the body fluid tester.

The test sheet 10 inserted into the body fluid tester according to an example of the present disclosure may be inserted into a sheet insertion hole of a carrier 110 and thereby inserted into the main body 100 of the body fluid tester as shown in (a) of FIG. 5. For reference, (a) of FIG. 5 shows a partial perspective view including a vertical section of the test sheet 10 and the carrier 110 for convenience of understanding.

The test sheet 10 according to the present disclosure may include a sheet body 13, a body fluid receiver 11, and a sheet electrode 12, as shown in FIG. 5B, and the sheet body 13, the body fluid receiver 11, and the sheet electrode 12 may be integrally formed. In the test sheet 10, a driver 150 may operate upon the sheet electrode 12 being inserted into the driver 150.

The sheet body 13 may serve to allow a user to grip the test sheet 10. The sheet body 13 may be integrally formed with the body fluid receiver 11 and the sheet electrode 12. In order for a user to easily distinguish the sheet body 13 from the body fluid receiver 11 and not to slip when gripping the sheet body 13, an anti-slip film may be attached to the sheet body 13 or an anti-slip coating layer may be formed in the sheet body 13.

The sheet body 13 may be formed of a transparent synthetic resin such as polyethylene and acrylic or may be formed of a material such as glass.

When the test sheet 10 is inserted into the driver 150, the sheet electrode 12 may be formed of a metal material to be electrically connected to an electrode terminal provided in the insertion hole of the driver 150.

To this end, a predetermined circuit pattern may be provided in the sheet electrode 12 for electrical connection with the circuit pattern formed in the driver 150. The circuit pattern of the sheet electrode 12 may be provided by forming a thin metal film in the sheet body 13 or by forming a conductive material such as carbon at an end of the sheet body 13.

The body fluid receiver 11 may be formed in a partial region of the sheet body 13 to receive a user's body fluid, and may be formed of a transparent material. A position of the body fluid receiver 11 may be determined to be located at a lens focal length of the lens unit 140.

The user may apply the body fluid thinly and uniformly to the body fluid receiver 11 by using a body fluid collecting means. The body fluid receiver 11 may be formed integrally with the seat body part 13 and may be formed to be transparent or translucent so that the body fluid can be imaged and checked. However, a specific solid color, for example, green and yellow, may be applied to obtain a clear image when imaging the body fluid, but the present disclosure is not necessarily limited thereto.

As shown in (b) of FIG. 5, the body fluid receiver 11 may be in a shape of a groove recessed from an upper surface of the test sheet 10. Or, as shown in (c) FIG. 5, the body fluid receiver 11 may as be configured such that a partition wall 11b is formed in an upper surface of the test sheet 10 and a groove shape 11a is provided within the partition wall 11b.

Here, as shown in (c) of FIG. 5, when the body fluid receiver 11 is formed with the partition wall in the upper surface of the test sheet 10, UV coating may be performed on the upper surface of the test sheet 10 to form the partition wall. A height and a width of the partition wall of the body fluid receiver 11 may be thinner than a thickness of the sheet body 13, for example.

As shown in (a) of FIG. 6, the test sheet 10 may be inserted into the main body 100 of the body fluid tester through the sheet insertion hole of the carrier 110.

Accordingly, as shown in (b) of FIG. 6, the test sheet 10 may be inserted into the driver 150.

To this end, the driver 150 may include an insertion module 150b, into which the end of the test sheet 10 is inserted, and a driving board 150a having a circuit pattern configured to emit light from the light source 170 as the end of the test sheet 10 is inserted.

The driving board 150a may serve as a structure for fixing the driver 150 to the inner case 130 and installing the light source 170 and the insertion module 150b.

The driving board 150a may have a shape corresponding to that of the power supply 160, and may be inserted into and coupled to the inner case 130.

In addition, although not shown in the driving board 150a of the driver 150, a separate conductive wire for electrical connection with the power supply 160 may be further included.

In addition, a circuit for electrically connecting the sheet insertion module 150b, the power supply 160, and the light source 170 may be configured in the driving board 150a of the driver 150.

Although an example in which the light source 170 is positioned above the driving board 150a and the insertion module 150b is positioned below the driving boarde 150a is illustrated, the present disclosure is not necessarily limited thereto.

In addition, the circuit formed in the driving board 150a may form an open circuit together with the insertion module 150b. When the test sheet 10 is inserted into the insertion module 150b, the open circuit may change to a closed circuit so that the driver 150 can operate.

Accordingly, when the test sheet 10 is not inserted into the insertion module 150b, the circuit provided in the driver 150 may be in a closed circuit state and may not operate.

In addition, when the test sheet 10 is inserted into the insertion module 150b, the closed circuit is completed and the driver 150 operates, and accordingly, the light source 170 positioned on the driver 150 may emit light.

To this end, the insertion module 150b of the driver 150 may include a switch for turning on when the test sheet 10 is inserted, or may include a separate terminal capable of contacting the test sheet 10.

In the present disclosure, since the test sheet 10 has been described as having the sheet electrode 12, the insertion module 150b may include a separate terminal electrically contacting the sheet electrode 12. However, the present disclosure is not necessarily limited thereto.

Accordingly, the insertion module 150b, together with the driving board 150a, may maintains an open circuit state when the test sheet 10 is not inserted, and when the test sheet 10 is inserted, the insertion module 150b may form a closed circuit by the test sheet 10 so that the driver 150 can operate.

Meanwhile, the carrier 110 of the body fluid tester according to the present disclosure may be inserted into the carrier hole H120 of the outer case 120 or may be easily separated from the carrier hole H120, and the upper and lower sides of the carrier 110 may be easily distinguished. Hereinafter, this will be described.

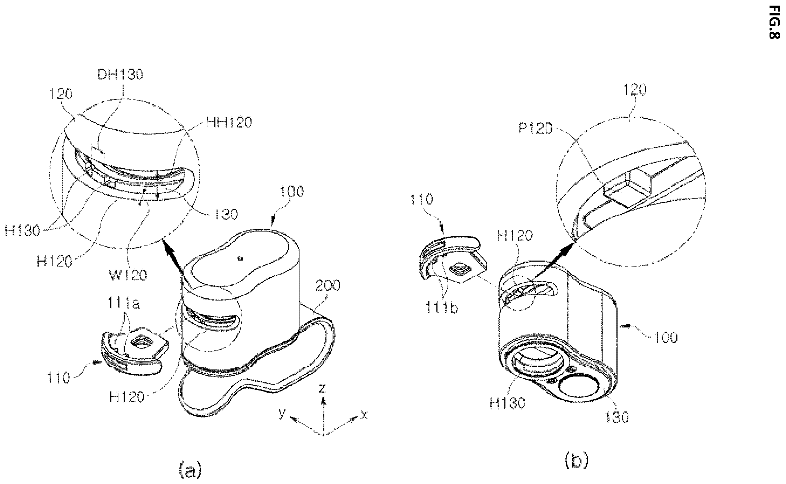

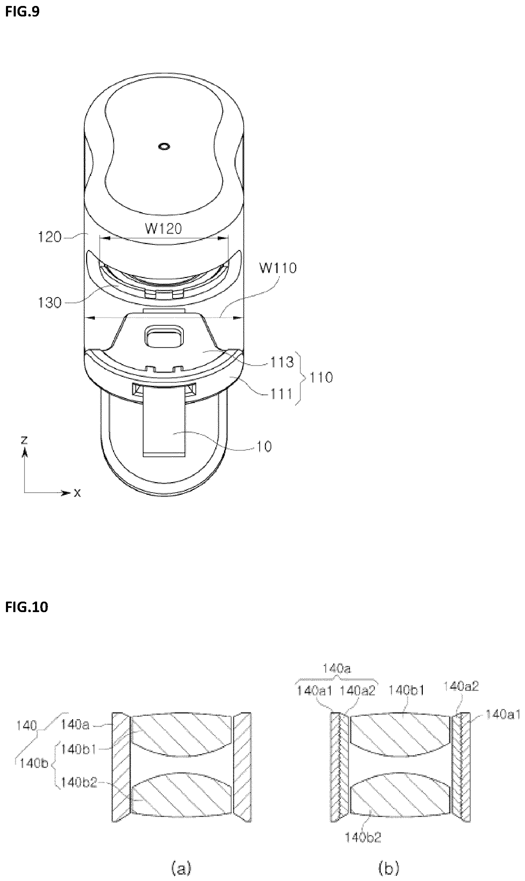

FIGS. 7A and 7B are diagrams for explaining in more detail the carrier 110 in the present disclosure, FIG. 8 is a view for explaining a coupling protrusion P110 of the outer case 120 to be coupled to the carrier 110 and the inner coupling hole of the inner case 130, and FIG. 9 is a view for comparision between a width of the carrier 110 in the second horizontal direction y and a width of the carrier hole H120 of the outer case 120 in the second horizontal direction y.

The carrier 110 according to an example of the present disclosure may include a head part 111 and a body part 113 as shown in (a) and (c) of FIG. 7A. As shown in (b) and (d) of FIG. 7, the carrier 110 may be formed such that the the sheet insertion hole 117 pass through the head part 111 and the body part 113 in the first horizontal direction x.

The head part 111 has a first thickness T111 and may be exposed from the front of the outer case 120. To this end, an outer shape of the head part 111 may include a curved shape like the outer case 120.

The first thickness T111 of the head part 111 may be smaller than a height of the carrier hole H120 provided in the outer case 120, and a width of the head part 111 in the first horizontal direction x may be greater than a height HH120 of the carrier hole H120 provided in the outer case 120.

The body part 113 has a second thickness T113 smaller than the first thickness T111 and may extend from the head part 111 in the first horizontal direction x, and a through hole 115 may be provided in the vertical direction z intersection the first and second horizontal directions. The width of the body portion 113 may decrease in the first horizontal direction x.

Due to the difference in thickness between the head part 111 and the body part 113 of the carrier 110, a first height difference H111a between the head part 111 and the body part 113 at an upper side of the carrier 110 and a second height difference H111b between the head 111 and the body 113 at a lower side of the carrier 110.

In (d) of FIG. 7A, an example in which the first height difference H111a and the second height difference H111b are identical to each other is illustrated. However, in order to distinguish the upper sisde and the lower side of the carrier 110, the first height difference H111a and the second height difference H111b may be different from each other. For example, the first height difference H111a may be smaller than the second height difference H111b. Accordingly, when the upper side and the lower side of the carrier 110 are turned upside down, the first height difference H111a and the second height difference H111b of the carrier 110 may become the opposite, preventing the carrier 110 from being inserted into the carrier hole H120 of the outer case 120.

This is because a height of the coupling protrusion P110 provided in the outer case 120 shown in FIG. 8 and a depth of the pair of inner coupling holes H130 provided in the inner case 130 are formed to match the first height difference H111a and the second height difference H111b, respectively.

A pair of upper protrusions 111a, as shown in (a) of FIG. 7A, and a pair of lower protrusions 111b, as shown in (c) of FIG. 7A, may be provided.

The pair of upper protrusions 111a may be positioned at the upper side of the carrier 110 and may protrude from the head part 111 of the carrier 110 toward the body part 113 in the first horizontal direction x. In addition, the pair of upper protrusions 111a may be spaced apart from each other at a first interval D111a and may have a first width W111a

The pair of lower protrusions 111b may be positioned at the lower side of the carrier 110 and may protrude from the head part 111 of the carrier 110 toward the body part 113 in the first horizontal direction x. In addition, the pair of lower protrusions 111b may be spaced apart from each other at a second interval D111b and may have a second width W111b.

Further, the carrier may be provided with coupling holes or coupling protrusions, the coupling holes into which the pair of upper protrusions 111a and the pair of lower protrusions 111b are inserted, and the coupling protrusions which are inserted between the pair of upper protrusions 111a and between the pair of lower protrusions 111b.

For example, a coupling protrusion P110 may be provided in the outer case 120 inside the carrier hole H120, as shown in (b) of FIG. 8, and a pair of inner coupling holes H130 may be provided in the inner case 130 inside the carrier hole H120, as shown in (a) of FIG. 8.

The coupling protrusion P110 provided in the outer case 120 may be positioned at an inner surface of the carrier hole H120 and protrude downward, and the coupling protrusion P110 may be inserted between the pair of the upper protrusions 111a provided in the carrier 110.

Here, a width of the coupling protrusion P110 provided in the outer case 120 may be smaller than a first interval D111a between the pair of upper protrusions 111a provided in the carrier 110.

The pair of inner coupling holes H130 provided in the inner case 130 may be positioned at a front upper end of the inner case 130, and the pair of lower protrusions 111b may be inserted thereinto. Here, an interval DH130 between the pair of inner coupling holes H130 may be smaller than the second interval D111b between the pair of lower protrusions 111b provided in the carrier 110, and a width of each of the inner coupling holes DH130 may be greater than a width of each of the lower protrusions 111b.

Accordingly, when the carrier 110 is inserted into the carrier hole H120 of the outer case 120, the head part 111 of the carrier 110 may be caught at the entrance of the carrier hole H120 to prevent the carrier 110 from being completely inserted into the carrier hole H120.

In addition, in order to distinguish the upper side and the lower side of the carrier 110, the first gap D111a between the pair of upper protrusions 111a may be different from the second gap D111b of the pair of lower protrusions 111b. Alternatively, the first width W111a of the pair of upper protrusions 111a may be different from the second width W111b of the pair of lower protrusions 111b.

Accordingly, a width of the coupling protrusion P110 provided in the outer case 120, a width of each of the inner coupling holes H130 provided in the inner case 130, and the interval between the inner coupling holes H130 may be different from one another.

Accordingly, when the upper and lower sides of the carrier 110 are turned upside down, the carrier 110 may be prevented from being properly inserted into the carrier hole H120.

In addition, as shown in FIG. 7B, a carrier horizontal stopper 119 protruding in the second horizontal direction y may be provided at a portion adjacent to the head part 111 in the body part 113 of the carrier 110, so that the carrier 110 cannot be naturally separated from the carrier hole H120 and a user can have a sense of feeling of insertion or separation when the carrier 110 is inserted into or separated from the carrier hole H120.

The carrier horizontal stopper 119 may be formed by coupling a metal plate with elasticity to the body part 113, as shown in (a) of FIG. 7B, or the body part 113 may form the carrier horizotal stopper 119 by itself. That is, a portion of the body part 113 adjacent to the head part 111 may protrude in a horizontal direction so that the horizontal stopper 119 may be formed integrally with the body part 113.

In addition, in order to prevent the carrier 110 from being completely inserted into the carrier hole H120, a maximum width W110 of the head part 111 of the carrier 110 in the second horizontal direction y may be greater than a minimum width W120 of the opening hole H120 of the outer case 120 in the second horizontal direction y, as shown in FIG. 9.

In addition, as described above, a width W111 of the head part 111 in the first horizontal direction x may be greater than a thickness W120 of the outer case 120 forming the carrier hole H120, so that the user can easily separate the carrier 110 from the carrier hole H120 of the outer case 120.

In the body fluid tester according to the present disclosure, it is possible for the user to arbitrarily adjust a focal length between the lens unit 140 and the test sheet 10 when the focal length is not appropriate. Hereinafter, this will be described.

FIG. 10 illustrates the lens unit 140 of the body fluid tester according to an example of the present disclosure in more detail.

As described above, the lens unit 140 according to an example of the present disclosure may be positioned at a front end of the inner case 130. As shown in (a) of FIG. 10, the lens unit 140 may include a lens housing 140a and a plurality of first and second lenses 140b 1 and 140b2.

The lens housing 140a may be formed in a cylindrical shape having a length in a vertical direction z. The first lens 140b1 may be positioned at an upper side of the lens housing 140a, and the second lens 140b2 may be spaced apart downward from the first lens 140b1 and positioned at a lower side of the lens housing 140a.

In general, a body fluid tester is manufactured and produced in a state in which the focal length between the lens unit 140 and the test sheet 10 is adjusted, but due to an error in a manufacturing process or an external impact occurring during a distribution process, the focal length between the lens unit 140 and the test sheet 10 may be varied.

Thus, in the present disclosure, in order to solve the aforementioned problem, the lens housing 140a may include an outer housing 140a1 and an inner housing 140a2, the outer housing 140a1 may have a screw groove formed in an inner surface thereof, and the inner housing 140a2 may have a screw groove formed in an outer surface thereof, the screw groove which is to be engaged with the screw groove of the outer housing 140a1, as shown in (b) of FIG. 10.

Accordingly, when the focal length of the lens is not appropriate, the user may rotate the inner housing 140a2 to vertically move the inner housing 140a2 within the outer housing 140a1 so as to adjust the focal length of the lens.

Although not illustrated, an adjust groove recessed upwards may be provided in a lower cylindrical edge section of the inner housing 140a2 in the cylindrical shape so as to facilitate the rotation of the rotation of the inner housing 140a2.

Accordingly, when the focal length between the lens unit 140 and the test sheet 10 is varied and an image of body fluid is not clear, the user may rotate the inner housing 140a2 within the outer housing 140a with a thing and long object, such as a pin or a needle, inserted into the adjust groove provided at the lower side of the inner housing 140ab, so that the user can adjust the length between the lens unit 140 and the test sheet 10 to adjust the focal length.

Accordingly, the convenience for use of the body fluid tester may be further improved.

In the body fluid tester according to an example of the present disclosure, some elements may improve, unlike the description provided with reference to FIGS. 3 and 4, so as to disperse light emitted from the light source 170 and easily recognize the light from the above of the body fluid tester. Hereinafter, this will be described.

FIG. 11 is a view for explaining a modified example in which a part of a body fluid tester according to the present disclosure shown in FIGS. 3 and 4 is changed.

In FIG. 11, any description redundant with the previously provided description will be omitted, and differences therefrom will be mainly described.

As shown in FIG. 11, in the body fluid tester according to a modified example of the present disclosure, a diffusing lens 173 for diffusing light emitted from the light source 170 may be further provided at an upper side of the light source 170.

Accordingly, the light emitted from the light source 170 may be uniformly diffused and thus radiated to the test sheet 10 through the reflection unit 180, thereby helping to acquire a clearer and improved image of the body fluid sample.

In addition, an upper light hole H120 through which the light emitted from the light source 170 passes may be further provided at an upper side of the outer case 120. That is, the upper light hole H120 through which the light emitted from the light source 170 passes may be provided in each of the first and second outer members of the outer case 120 provided above the diffusing lens 173 positioned on the top of the light source 170.

Accordingly, after the light emitted from the light source 170 is diffused by the diffusing lens 173, some of the light may be incident onto the test sheet 10 through the reflection unit 180 and the remaining light may be transmitted to the outside of the main body 100 through the upper light hole H120 provided in the outer case 120.

Accordingly, the user may be able to more conveniently and easily recognize whether the light source 170 inside the main body 100 is operating properly.

FIG. 12 is a view for explaining the main body fixing part 200 in the body fluid tester according to an example of the present disclosure.

As shown in FIG. 12, the main body fixing part 200 may have a clip shape with elasticity, and may include a recessed groove detachably attached to a bottom of the inner case 130.

The main body fixing part 200 may be bent at a plurality of point, and may be formed of a material having a restoration force due to elasticity, such as synthetic resin or metal.

The fixing part observation hole H200 may be provided in a surface in contact with the observation opening hole of the inner case 130 in a bottom of a recessed groove of the main body fixing part 200, and a magnetic body 210 may be provided in at least a portion of the remaining part of the bottom of the recessed groove.

The lens unit 140 of the main body 100 and the camera of the user terminal 1 may face each other through the fixing part observation hole H200 provided in the main body fixing part 200.

In addition, due to an attractive force between the magnetic body 210 of the main body fixing part 200 and the metal member 131 provided in the inner case 130, the main body 100 and the main body fixing part 200 may be detachably attached to each other.

As such, in the body fluid tester according to the present disclosure, it is possible to easily separate the carrier 110 from the main body 100, adjust the focal length of the lens unit 140 provided in the main body 100, easily check whether the light source 170 operates from the above of the main body 100 and easily separate and attach the case and the main body 100, thereby improving convenience of use.

While the present disclosure has been shown and described with reference to exemplary embodiments thereof, it will be apparent to those of ordinary skill in the art that various changes in form and detail may be made thereto without departing from the scope of the present disclosure as defined by the following claims. Accordingly, such modifications are deemed to be within the scope of the present disclosure, and the scope of the present disclosure is determined by the appended claims.

## Claims

1. A body fluid tester (100) comprising:
a carrier (110) having a sheet insertion hole (117), the sheet insertion hole being configured to receive a test sheet (10) by insertion in a first horizontal direction;
an outer case (120) having a carrier hole (H120) formed in a front side thereof, the carrier hole (H120)
which has its length in a second horizontal direction perpendicular to the first horizontal direction and intersecting with the first horizontal direction and into which the carrier (110) is configured to be inserted, the outer case (120) having an opened lower-side (123);
an inner case (130) configured to be inserted upwards-through the opened lower-side of the outer case (120) following a vertical direction perpendicular to the horizontal directions to be coupled thereto positioned at an inner surface of the outer case (120), and having a case observation hole (H130) at a bottom side thereof; and
a lens unit (140) positioned on a side of an inner front end of the inner case (130) and positioned between the carrier (110) and the case observation hole (H130), wherein the carrier (110) is configured to be inserted into or separated from the carrier hole (H120) of the outer case (120),
wherein the carrier (110) comprises:
a head part (111) having a first thickness and configured to be exposed to a front of the outer case (120); and
a body part (113) having a second thickness smaller than the first thickness, extending from the head part in the first horizontal direction , and having a through hole (115) opened in the vertical direction intersecting with the first and second horizontal directions, wherein the through hole (115) of the carrier (110), the lens unit (140), and the case observation hole (131) of the inner case (130) are configured to be aligned along a vertical optical path when the carrier (110) is fully inserted into the outer case (120).

2. The body fluid tester of claim 1, wherein a maximum width of the head part (111) in the second horizontal direction is greater than a minimum width of an opening hole of the outer case in the second horizontal direction.

3. The body fluid tester of claim 1, wherein the body part (113) comprises:
a pair of upper protrusions (111a) provided at an upper side of the body part, protruding from the head of the carrier in the first horizontal direction, spaced apart from each other at a first interval, and having a first width (W111a); and
a pair of lower protrusions (111b) provided at a lower side of the body part, protruding from the head part of the carrier in the first horizontal direction, spaced apart from each other at a second interval, and having a second width (W111b).

4. The body fluid tester of claim 3, wherein:
a coupling protrusion (P110) is provided in an inner surface of the outer case, the coupling protrusion protruding downward to be inserted between the pair of the upper protrusions (111a), and
a pair of inner coupling holes (H130) is provided at a front upper end of the inner case, the inner coupling holes into which the pair of lower protrusions are to be inserted.

5. The body fluid tester of claim 4, wherein a first height difference (H111a) between the head part and the body part at an upper side of the carrier is different from a second height difference (H111b) between the head part and the body part at a lower side of the carrier.

6. The body fluid tester of claim 4, wherein the first interval (D111a) between the pair of upper protrusions is different from the second interval (D111b) between the pair of lower protrusions, or a first width between the pair of upper protrusions is different from a second width between the pair of lower protrusions.

7. The body fluid tester of claim 1, wherein the lens unit (140) comprises:
an outer housing (140a) having a length in a vertical direction (z); and
an inner housing (140b) positioned within the outer housing, having a length in the vertical direction (z), and having a plurality of lenses spaced apart from each other in the vertical direction, and wherein the outer housing has a screw groove provided in an inner surface thereof,
wherein the inner housing has a screw groove provided in an outer surface thereof to be engaged with the screw groove of the outer housing (140a).

8. The body fluid tester of claim 1, further comprising:
a light source (170) provided above the carrier (110) within the outer case (120); and
a diffusing lens (173) provided above the light source to diffuse light emitted from the light source.

9. The body fluid tester of claim 8, further comprising:
an upper light hole (H120) provided at an upper side of the outer case, the upper light hole through which the light emitted from the light source passes.

10. The body fluid tester of claim 1, wherein:
the test sheet (10) comprises a body fluid receiver, and
the body fluid receiver (11) is provided in a shape of a groove recessed from an upper side of the test sheet, or the body fluid receiver is provided by forming a partition wall (11b) at the upper surface of the test sheet.

11. The body fluid tester of claim 1, further comprising:
a main body fixing part (200) separable from a bottom of the inner case and having a recessed groove,
wherein a fixing part observation hole (H200) is provided in a surface in contact with a case observation hole (H130) of the inner case (130) in a bottom of the recessed groove and a magnetic body (210) is provided in at least a portion of the remaining part of the bottom of the recessed groove.

## Patentansprüche

1. Körperfluidtester (100), umfassend:
einen Träger (110), der ein Streifeneinführloch (117) aufweist, wobei das Streifeneinführloch dazu konfiguriert ist, einen Teststreifen (10) durch Einführen in eine erste horizontale Richtung aufzunehmen;
ein äußeres Gehäuse (120), in dessen Vorderseite ein Trägerloch (H120) ausgebildet ist, wobei das Trägerloch (H120), das seine Länge in einer zweiten horizontalen Richtung senkrecht zu der ersten horizontalen Richtung aufweist, die sich mit der ersten horizontalen Richtung schneidet, und in das der Träger (110) dazu konfiguriert ist, eingesetzt zu werden, wobei das äußere Gehäuse (120) eine geöffnete Unterseite (123) aufweist;
ein inneres Gehäuse (130), das dazu konfiguriert ist, nach oben durch die geöffnete Unterseite des äußeren Gehäuses (120), einer vertikalen Richtung folgend, senkrecht zu den horizontalen Richtungen eingeführt zu werden, um daran, an einer inneren Fläche des äußeren Gehäuses (120) positioniert gekoppelt zu werden, und ein Gehäusebeobachtungsloch (H130) an einer Unterseite davon aufweist; und
eine Linseneinheit (140), die an einer Seite eines inneren vorderen Endes des inneren Gehäuses (130) positioniert und zwischen dem Träger (110) und dem Gehäusebeobachtungsloch (H130) positioniert ist, wobei der Träger (110) dazu konfiguriert ist, in das Trägerloch (H120) des äußeren Gehäuses (120) eingesetzt oder davon getrennt zu werden,
wobei der Träger (110) Folgendes umfasst:
einen Kopfteil (111), der eine erste Dicke aufweist und dazu konfiguriert ist, einer Vorderseite des äußeren Gehäuses (120) ausgesetzt zu sein; und
einen Körperteil (113), der eine zweite Dicke aufweist, die kleiner als die erste Dicke ist, der sich von dem Kopfteil in der ersten horizontalen Richtung erstreckt und ein Durchgangsloch (115) aufweist, das in der vertikalen Richtung geöffnet ist, die sich mit der ersten und zweiten horizontalen Richtung schneidet, wobei das Durchgangsloch (115) des Trägers (110), die Linseneinheit (140) und das Gehäusebeobachtungsloch (131) des inneren Gehäuses (130) dazu konfiguriert sind, entlang eines vertikalen optischen Pfads ausgerichtet zu sein, wenn der Träger (110) vollständig in das äußere Gehäuse (120) eingesetzt ist.

2. Körperfluidtester nach Anspruch 1, wobei eine maximale Breite des Kopfteils (111) in der zweiten horizontalen Richtung größer als eine minimale Breite eines Öffnungslochs des äußeren Gehäuses in der zweiten horizontalen Richtung ist.

3. Körperfluidtester nach Anspruch 1, wobei der Körperteil (113) Folgendes umfasst:
ein Paar oberer Vorsprünge (111a), die an einer oberen Seite des Körperteils bereitgestellt sind, von dem Kopf des Trägers in der ersten horizontalen Richtung vorstehen, in einem ersten Abstand voneinander beabstandet sind und eine erste Breite (W111a) aufweisen; und
ein Paar unterer Vorsprünge (111b), die an einer unteren Seite des Körperteils bereitgestellt sind, von dem Kopfteil des Trägers in der ersten horizontalen Richtung vorstehen, in einem zweiten Abstand voneinander beabstandet sind und eine zweite Breite (W111b) aufweisen.

4. Körperfluidtester nach Anspruch 3, wobei:
ein Kopplungsvorsprung (P110) in einer inneren Fläche des äußeren Gehäuses bereitgestellt ist, wobei der Kopplungsvorsprung nach unten vorsteht, um zwischen das Paar der oberen Vorsprünge (111a) eingeführt zu werden, und
ein Paar innerer Kopplungslöcher (H130) an einem vorderen oberen Ende des inneren Gehäuses bereitgestellt ist, wobei die inneren Kopplungslöcher, in die das Paar unterer Vorsprünge eingesetzt werden soll.

5. Körperfluidtester nach Anspruch 4, wobei sich eine erste Höhendifferenz (H111a) zwischen dem Kopfteil und dem Körperteil an einer oberen Seite des Trägers von einer zweiten Höhendifferenz (H111b) zwischen dem Kopfteil und dem Körperteil an einer unteren Seite des Trägers unterscheidet.

6. Körperfluidtester nach Anspruch 4, wobei sich der erste Abstand (D111a) zwischen dem Paar oberer Vorsprünge von dem zweiten Abstand (D111b) zwischen dem Paar unterer Vorsprünge unterscheidet oder sich eine erste Breite zwischen dem Paar oberer Vorsprünge von einer zweiten Breite zwischen dem Paar unterer Vorsprünge unterscheidet.

7. Körperfluidtester nach Anspruch 1, wobei die Linseneinheit (140) Folgendes umfasst:
ein äußeres Gehäuse (140a), das eine Länge in einer vertikalen Richtung (z) aufweist; und
ein inneres Gehäuse (140b), das innerhalb des äußeren Gehäuses positioniert ist, eine Länge in der vertikalen Richtung (z) aufweist und eine Vielzahl von Linsen aufweist, die in der vertikalen Richtung voneinander beabstandet sind, und wobei das äußere Gehäuse eine Schraubennut aufweist, die in einer inneren Fläche davon bereitgestellt ist,
wobei das innere Gehäuse eine Schraubennut aufweist, die in einer äußeren Fläche davon bereitgestellt ist, um mit der Schraubennut des äußeren Gehäuses (140a) in Eingriff zu kommen.

8. Körperfluidtester nach Anspruch 1, ferner umfassend:
eine Lichtquelle (170), die über dem Träger (110) innerhalb des äußeren Gehäuses (120) bereitgestellt ist; und
eine Streulinse (173), die über der Lichtquelle bereitgestellt ist, um Licht, das von der Lichtquelle emittiert wird, zu streuen.

9. Körperfluidtester nach Anspruch 8, ferner umfassend:
ein oberes Lichtloch (H120), das an einer oberen Seite des äußeren Gehäuses bereitgestellt ist, wobei das obere Lichtloch, durch das das von der Lichtquelle emittierte Licht hindurchtritt.

10. Körperfluidtester nach Anspruch 1, wobei:
der Teststreifen (10) eine Körperfluidaufnahme umfasst, und
die Körperfluidaufnahme (11) in einer Form einer Nut bereitgestellt ist, die von einer oberen Seite des Teststreifens ausgespart ist, oder die Körperfluidaufnahme durch Bilden einer Trennwand (11b) an der oberen Fläche des Teststreifens bereitgestellt ist.

11. Körperfluidtester nach Anspruch 1, ferner umfassend:
ein Hauptkörperbefestigungsteil (200), das von einem Boden des inneren Gehäuses trennbar ist und eine ausgesparte Nut aufweist,
wobei ein Befestigungsteilbeobachtungsloch (H200) in einer Fläche in Kontakt mit einem Gehäusebeobachtungsloch (H130) des inneren Gehäuses (130) in einem Boden der ausgesparten Nut bereitgestellt ist und ein Magnetkörper (210) in mindestens einem Abschnitt des verbleibenden Teils des Bodens der ausgesparten Nut bereitgestellt ist.

## Revendications

1. Testeur de liquide corporel (100), comprenant :
un support (110) comportant un trou d'insertion de feuille (117), le trou d'insertion de feuille étant conçu pour recevoir une feuille de test (10) par insertion dans une première direction horizontale ;
un boîtier externe (120) comportant un trou de support (H120) formé dans un côté avant de celui-ci, le trou de support (H120) qui a sa longueur dans une seconde direction horizontale perpendiculaire à la première direction horizontale et croisant la première direction horizontale et dans laquelle le support (110) est conçu pour être inséré, le boîtier externe (120) possédant un côté inférieur ouvert (123) ;
un boîtier interne (130) conçu pour être inséré vers le haut à travers le côté inférieur ouvert du boîtier externe (120) suivant une direction verticale perpendiculaire aux directions horizontales de manière à y être couplé positionné au niveau d'une surface interne du boîtier externe (120), et comportant un trou d'observation de boîtier (H130) au niveau d'un côté inférieur de celui-ci ; et
une unité de lentille (140) positionnée sur un côté d'une extrémité avant interne du boîtier interne (130) et positionnée entre le support (110) et le trou d'observation de boîtier (H130), ledit support (110) étant configuré pour être inséré dans le trou de support (H120) du boîtier externe (120) ou séparé de celui-ci,
ledit support (110) comprenant :
une partie tête (111) présentant une première épaisseur et conçue pour être exposée à l'avant du boîtier externe (120) ; et
une partie corps (113) présentant une seconde épaisseur plus petite que la première épaisseur, s'étendant à partir de la partie tête dans la première direction horizontale, et comportant un trou traversant (115) ouvert dans la direction verticale croisant les première et seconde directions horizontales, ledit trou traversant (115) du support (110), ladite unité de lentille (140) et ledit trou d'observation de boîtier (131) du boîtier interne (130) étant conçus pour être alignés le long d'un chemin optique vertical lorsque le support (110) est complètement inséré dans le boîtier externe (120).

2. Testeur de liquide corporel selon la revendication 1, une largeur maximale de la partie tête (111) dans la seconde direction horizontale étant supérieure à une largeur minimale d'un trou d'ouverture du boîtier externe dans la seconde direction horizontale.

3. Testeur de liquide corporel selon la revendication 1, ladite partie corps (113) comprenant :
une paire de saillies supérieures (111a) ménagées sur un côté supérieur de la partie corps, faisant saillie de la tête du support dans la première direction horizontale, espacées l'une de l'autre à un premier intervalle, et présentant une première largeur (W111a) ; et
une paire de saillies inférieures (111b) ménagées sur un côté inférieur de la partie corps, faisant saillie de la partie tête du support dans la première direction horizontale, espacées l'une de l'autre à un second intervalle, et présentant une seconde largeur (W111b).

4. Testeur de liquide corporel selon la revendication 3 :
une saillie de couplage (P110) étant ménagée dans une surface interne du boîtier externe, la saillie de couplage faisant saillie vers le bas de manière à être insérée entre la paire de saillies supérieures (111a), et
une paire de trous de couplage internes (H130) étant ménagée au niveau d'une extrémité supérieure avant du boîtier interne, les trous de couplage internes dans lesquels la paire de saillies inférieures doivent être insérées.

5. Testeur de liquide corporel selon la revendication 4, la première différence de hauteur (H111a) entre la partie tête et la partie corps au niveau d'un côté supérieur du support étant différente de la seconde différence de hauteur (H111b) entre la partie tête et la partie corps au niveau d'un côté inférieur du support.

6. Testeur de liquide corporel selon la revendication 4, ledit premier intervalle (D111a) entre la paire de saillies supérieures étant différent du second intervalle (D111b) entre la paire de saillies inférieures, ou une première largeur entre la paire de saillies supérieures étant différente de la seconde largeur entre la paire de saillies inférieures.

7. Testeur de liquide corporel selon la revendication 1, ladite unité de lentille (140) comprenant :
un logement externe (140a) présentant une longueur dans une direction verticale (z) ; et
un logement interne (140b) positionné à l'intérieur du logement externe, présentant une longueur dans la direction verticale (z), et comportant une pluralité de lentilles espacées les unes des autres dans la direction verticale, et ledit logement externe comportant une rainure de vis prévue dans une surface interne de celui-ci,
ledit logement interne comportant une rainure de vis ménagée dans une surface externe de celui-ci destinée à être mise en prise avec la rainure de vis du logement externe (140a).

8. Testeur de liquide corporel selon la revendication 1, comprenant en outre :
une source de lumière (170) disposée au-dessus du support (110) à l'intérieur du boîtier externe (120) ; et
une lentille de diffusion (173) disposée au-dessus de la source de lumière destinée à diffuser la lumière émise par la source de lumière.

9. Testeur de liquide corporel selon la revendication 8, comprenant en outre :
un trou de lumière supérieur (H120) ménagé sur un côté supérieur du boîtier externe, le trou de lumière supérieur à travers lequel passe la lumière émise à partir de la source de lumière.

10. Testeur de liquide corporel selon la revendication 1 :
ladite feuille de test (10) comprenant un récepteur de liquide corporel, et
ledit récepteur de liquide corporel (11) étant pourvu sous une forme d'une rainure en retrait par rapport à un côté supérieur de la feuille de test, ou ledit récepteur de liquide corporel étant pourvu en formant une paroi de séparation (11b) au niveau de la surface supérieure de la feuille de test.

11. Testeur de liquide corporel selon la revendication 1, comprenant en outre :
une partie de fixation de corps principal (200) séparable du fond du boîtier interne et comportant une rainure en retrait,
un trou d'observation de partie de fixation (H200) étant ménagé dans une surface en contact avec un trou d'observation de boîtier (H130) du boîtier interne (130) dans le fond de la rainure évidée et un corps magnétique (210) étant disposé dans au moins une partie de la partie restante du fond de la rainure en retrait.
